Europäisches Patentamt

(19) European Patent Office

Office européen des brevets

(11) Veröffentlichungsnummer: **0 120 199**
**B1**

(12) **EUROPÄISCHE PATENTSCHRIFT**

(45) Veröffentlichungstag der Patentschrift:
18.12.85

(51) Int. Cl.⁴: **C 07 C 63/16, C 07 C 51/573**

(21) Anmeldenummer: **84100493.0**

(22) Anmeldetag: **18.01.84**

(54) **Verfahren zur Entfernung von Naphthochinon aus durch Luftoxidation von Naphthalin hergestelltem Phthalsäureanhydrid.**

(30) Priorität: **16.03.83 DE 3309310**
**11.08.83 DE 3329026**

(43) Veröffentlichungstag der Anmeldung:
**03.10.84 Patentblatt 84/40**

(45) Bekanntmachung des Hinweises auf die Patenterteilung:
**18.12.85 Patentblatt 85/51**

(84) Benannte Vertragsstaaten:
**AT BE DE FR GB IT NL SE**

(56) Entgegenhaltungen:
**DE - A - 1 618 874**
**US - A - 1 728 225**
**US - A - 2 510 852**

(73) Patentinhaber: **HÜLS AKTIENGESELLSCHAFT,**
**Patentabteilung / PB 15 - Postfach 13 20,**
**D-4370 Marl 1 (DE)**

(72) Erfinder: **Gude, Fritz, Dr., Wilhelmstrasse 4,**
**D-4690 Herne 2 (DE)**
Erfinder: **Haferkorn, Herbert, Horster Strasse 528,**
**D-4250 Bottrop (DE)**

## Beschreibung

Das durch katalytische Luftoxidation von Naphthalin gewonnene Rohphthalsäureanhydrid enthält neben den durch Destillation leicht abtrennbaren Nebenprodukten, wie z.B. Maleinsäureanhydrid, Schwefel usw., noch i. a. mehr als 0,5 Gew.-% Naphthochinon. Da dieses im Siedebereich des Phthalsäureanhydrids liegt und deshalb Farbe und Reinheit des Phthalsäureanhydrids vermindert, muss es vor der Destillation entfernt werden. Danach soll der Naphthochinongehalt im raffinierten Rohprodukt weniger als 5 ppm betragen. Das Destillat, also das Reinphthalsäureanhydrid, darf eine Farbzahl nach ASTM-Norm D 1209 von maximal 10 APHA und einen Naphthochinongehalt von maximal 1 ppm aufweisen. Im allgemeinen geschieht die Entfernung des Naphthochinons durch Überführung in höhere Kondensationsprodukte, die dann bei der Destillation des Rohphthalsäureanhydrids im Rückstand verbleiben. Es sind mehrere Katalysatoren für diese Kondensation vorgeschlagen worden, die aber alle grosse Nachteile aufweisen. So wird in der US-PS Nr. 2356499 Zinn-II-chlorid mit Natriumcarbonat vorgeschlagen. Die US-PS Nr. 2855440 empfiehlt eine Kombination von Schwefel- und Borsäure und die US-PS Nr. 2670325 Alkalihydroxid. Nach Ullmanns „Encyclopädie der Technischen Chemie", 3. Aufl., 13. Bd., S. 721, verwendet man hauptsächlich sogar konzentrierte Schwefelsäure bei einer Temperatur von rund 200° C.

Es ist aus der Praxis bekannt, dass die Schwefelsäure unter den Reaktionsbedingungen die Reaktorwände angreift und saure Gase abspaltet. Die anschliessend übliche Neutralisation der Schwefelsäure im Rohphthalsäureanhydrid mit Kreide führt zu anorganischen Salzen, die sich durch Absetzungen in den Destillationsblasen und die dadurch bedingte Behinderung der Destillation sehr negativ bemerkbar machen. Alkalien enthaltende Substanzen dagegen katalysieren in den vorgeschlagenen hohen Konzentrationen die Kohlendioxidabspaltung aus der Phthalsäure und senken so die Ausbeute an Phthalsäureanhydrid erheblich. Eigene Versuche, das Naphthochinon im Phthalsäureanhydrid an ungesättigte natürliche Öle, wie z. B. Kokosöl, Holzöl, ungesättigte natürliche freie Fettsäure usw. zu binden, scheiterten an ihrer geringen Aktivität. Einzig Leinöl senkte den Naphthochinonwert auf etwa 5 ppm, doch konnte durch die nachfolgende Vakuumdestillation nur stark gefärbtes Phthalsäureanhydrid gewonnen werden.

Gemäss DE-OS Nr. 1618874 soll es zwar möglich sein, mit derartigen Naturstoffen ein Phthalsäureanhydrid mit sehr geringen APHA-Einheiten zu erhalten, wahrscheinlich handelt es sich bei den Zusätzen aber um aufwendig vorgereinigte Einsatzstoffe.

Wie sich nun überraschend herausstellte, können geringe Mengen mehrere Doppelbindungen im Molekül enthaltende, durch Polymerisation von Butadien, Isopren, Dimethylbutadien, 1,3-Pentadien oder Gemische dieser Substanzen erhaltene Öle im Molgewichtsbereich von 800-5000 – bevorzugt 1000-3000 – sehr wirksam zur Entfernung von Naphthochinon aus Rohphthalsäureanhydrid unter den Reaktionsbedingungen verwendet werden.

Ein aus Naphthalin hergestelltes Rohphthalsäureanhydrid besass 0,7-0,8 Gew.-% Naphthochinon und 0,04 Gew.-% Maleinsäureanhydrid. Überraschenderweise gelang es, durch 20stündiges Erhitzen mit z. B. 0,2 Gew.-% handelsüblichen, isolierte Doppelbindungen enthaltenden Polybutadienölen mit den erfindungsgemässen Molgewichten auf annähernd 240° C nach Vakuumdestillation ein farbloses Reinphthalsäureanhydrid mit dem Schmelzpunkt von 131,2° C, einem Naphthochinongehalt von unter 1 ppm und einer Farbzahl von unter 10 APHA zu erhalten. Ähnliche Ergebnisse konnten auch beim Einsatz von Polyisopren, Polydimethylbutadien und Poly-1,3-Pentadien gefunden werden. Das gleiche Rohphthalsäureanhydrid wurde 8 h mit nur 0,1% nach z.B. DE-OS Nrn. 2924598, 2924577 oder E- PS Nr. 20924 isomerisiertem Polyöl (M=1500) auf 240° C erhitzt. Nach der Reaktion und Destillation war der Naphthochinongehalt unerwartet und überraschend unter 1 ppm gefallen. Bezogen auf den Gehalt an 1,3-Diendoppelbindungen hätte bei der Annahme einer Diels-Alder-Addition die 20fache Menge an isomerisiertem Polybutadienöl zur Bindung des Naphthochinons eingesetzt werden müssen. Die anschliessende Vakuumdestillation erbrachte eine Farbzahl von unter 10 APHA.

Praktisch gleiche Ergebnisse konnten bei Verwendungen von isomerisiertem Polyöl mit dem Molgewicht von etwa 3000, bei isomerisiertem Polyisopren (M=1300) und isomerisiertem Polypiperylen (M=1400) gefunden werden.

Wir stellten nun weiter fest, dass der Gehalt an Maleinsäureanhydrid im rohen Phthalsäureanhydrid die Wirkung der Dienpolymerisate herabsetzt. Wie bekannt, kann man eine Zersetzung des Maleinsäureanhydrids mit katalytischen Mengen alkalischer Substanzen durchführen (siehe z.B. Kirk-Othmer, Sec. Ed., vol. 12, S. 822).

Wie sich nun weiterhin überraschend herausstellte, lässt sich die notwendige Dosis an Dienpolymerisat ohne Nachteile reduzieren, wenn man der Phthalsäureanhydridschmelze zusätzlich eine geringe Menge Alkaliionen, z. B. in Form der Hydroxyde, Carbonate, Bicarbonate, Alkoholate, Bisulfite usw. –, oder auch Gemische dieser Verbindungen –, zusetzt. Diese Substanzen können auch in Polyöl gelöst sein. In erster Linie kommen Natrium- oder Kalium-, aber auch Lithiumverbindungen in Betracht. Beste Ergebnisse werden erreicht, wenn die Alkaliverbindung mit dem Dienpolymerisat gleichzeitig dosiert wird. Da ein Alkalizusatz auch die Zersetzung des Phthalsäureanhydrids begünstigt, sollte zur Vermeidung von merklichen Ausbeuteverlusten nur sehr vorsichtig dosiert werden. Es genügt die katalytisch notwendige Menge zur Zersetzung des Maleinsäureanhydrids, also etwa 1-40 ppm Alkaliionen bezogen auf das rohe Phthalsäureanhydrid, also erheblich geringere Mengen als zur Zersetzung des Naphthochinons

in Phthalsäureanhydrid vorgeschlagen werden. Die Reduzierung der Polydienmenge kommt trotzdem voll zur Wirkung.

Gegenstand der Erfindung ist daher ein Verfahren zur Entfernung von Naphthochinon aus durch Luftoxidation von Naphthalin oder Naphthalin enthaltenden Gemischen hergestelltem Phthalsäureanhydrid, das dadurch gekennzeichnet ist, dass das Naphthochinon enthaltende Rohphthalsäureanhydrid bei einer Temperatur von 180-280° C mit einem ungesättigten aliphatischen, durch Polymerisation von 1,3-Dienen hergestellten Öl vom Molgewicht 800-5000 — bevorzugt 1000-3000 — behandelt wird, ggf. in Gegenwart von 1-40 ppm — vorzugsweise 2-20 ppm — Alkaliionen, bezogen auf Rohphthalsäureanhydrid.

*Beispiele*

A) Behandlung von Rohphthalsäureanhydrid mit Polydienen.

1) 500 g Rohphthalsäureanhydrid, hergestellt durch Naphthalin-Gasphase-Oxidation, enthielt 0,67 Gew.-% Naphthochinon (NCh) und 0,64 Gew.-% Maleinsäureanhydrid (MSA). Diese Probe wurde aufgeschmolzen, mit 0,5 g (=0,1%)

Polybutadien mit dem Molekulargewicht von ca. 1500, das durch Isomerisierung unter dem katalytischen Einfluss von Alkali etwa 19% der Doppelbindungen in konjugierter Stellung aufwies, versetzt und während 7 h auf 270° C erhitzt. Nach dieser Behandlung betrug der NCh-Gehalt weniger als 5 mg/kg.

2) Vergleichsversuch: 370 g Rohphthalsäureanhydrid gleicher Herkunft mit einem NCh-Gehalt von 0,66 Gew.-% und 0,04 Gew.-% MSA wurde ohne Zusätze während 8 h auf 270° C gehalten. Nach dieser Behandlung wurde ein NCh-Gehalt von 0,40 Gew.-% analysiert. Die allein thermische Behandlung hat nur geringen Einfluss auf den NCh-Gehalt.

3 bis 9) Jeweils 50 g Rohphthalsäureanhydrid mit einem NCh-Gehalt von 0,69 Gew.-% wurden in Testrohren mit folgenden Zusätzen versehen und 20 h auf 270° C erhitzt: Polybutadien (Mol-Gew. ca. 1500), Polybutadien (Mol-Gew. ca. 3000), Polypiperylen (Mol-Gew. ca. 2500), Polyisopren (Mol-Gew. ca. 2000), Isomerginsäure der Fa. Harburger Fettchemie Brinkmann & Mergell GmbH (konjugierte natürliche Fettsäuren), Kokosöl, Leinöl. Nach der Behandlung wurden folgende Restgehalte an NCh bestimmt:

| Zusatz: 0,2% Polybutadien | (1500) | Restgeh.NCh <5 ppm |
|---|---|---|
| 0,2% Polybutadien | (2000) | Restgeh.NCh <5 ppm |
| 0,3% Polypiperylen | (2500) | Restgeh.NCh <5 ppm |
| 0,3% Polyisopren | (2500) | Restgeh.NCh <5 ppm |
| 0,2% Isomerginsäure | | Restgeh.NCh 17 ppm |
| 0,2% Kokosöl | | Restgeh.NCh 0,24% |
| 0,2% Leinöl | | Restgeh.NCh <5 ppm |

10) 500 g Rohphthalsäureanhydrid, hergestellt durch Gasphase-Oxidation eines Naphthalin/o-Xylol-Gemisches, enthielt 0,32 Gew.-% NCh und 0,8 Gew.-% MSA. Diese Probe wurde aufgeschmolzen, mit 0,5 g (=0,1 Gew.-%) Polybutadien mit dem Molekulargewicht von ca. 1500, das durch Isomerisierung etwa 12% der Doppelbindungen in konjugierter Stellung aufwies, versetzt und während 7 h auf 270° C erhitzt. Nach dieser Behandlung war der NCh-Gehalt auf 25 ppm gesenkt worden.

11) 500 g Rohphthalsäureanhydrid, hergestellt durch Gasphase-Oxidation eines Naphthalin/o-Xylol-Gemisches, enthielt 0,32 Gew.-% NCh und 0,8 Gew.-% MSA. Diese Probe wurde aufgeschmolzen und 4 h mit 30 ppm wasserfreie Soda auf 270° C erhitzt. Anschliessend wurde mit 0,5 g Polybutadien der unter 10 beschriebenen Qualität versetzt und 7 h bei gleicher Temperatur weiter erhitzt. Danach betrug der NCh-Gehalt 6 ppm.

12) 500 g Rohphthalsäureanhydrid, hergestellt durch Gasphase-Oxidation eines Naphthalin/o-Xylol-Gemisches, enthielt 0,32 Gew.-% NCh mit 0,8 Gew.-% MSA. Diese Probe wurde aufgeschmolzen und 7 h mit 0,5 g Polybutadienöl obiger Qualität und zusammen mit 30 ppm wasserfreie Soda auf 270° C erhitzt. Danach war der NCh-Gehalt unter 5 ppm gefallen.

B) Gewinnung von Reinphthalsäureanhydrid durch Destillation von raffiniertem Rohphthalsäureanhydrid.

13) Das nach Vers. 1 raffinierte Rohphthalsäureanhydrid (NCh-Gehalt <5 ppm) wurde bei 80 mbar über eine 10bödige Siebbodenkolonne bei einem Rücklaufverhältnis 2:1 destilliert. Das Destillat wies einen Schmelzpunkt von 131,2° C auf, eine Farbzahl von <10 APHA und einen NCh-Gehalt von <1 ppm.

14) Die mit Polybutadien, Polypiperylen und Polyisopren (Versuche 3 bis 6) raffinierten Rohphthalsäureanhydride wurden unter gleichen Bedingungen wie in Versuch 13 destilliert. Die Destillate wiesen ebenfalls einen Schmelzpunkt von 131,2° C, eine Farbzahl von <10 APHA und einen NCh-Gehalt von <1 ppm auf.

15) Vergleichsversuch: Ein mit Leinöl raffiniertes Rohphthalsäureanhydrid (NCh-Gehalt <5 ppm) wurde unter den Versuchsbedingungen, wie in Versuch 13 beschrieben, destilliert. Das anfallende Destillat war stark verfärbt.

**Patentanspruch**

Verfahren zur Entfernung von Naphthochinon aus durch Luftoxidation von Naphthalin oder Naphthalin enthaltenden Gemischen hergestelltem Phthalsäureanhydrid, dadurch gekennzeichnet, dass das Naphthochinon enthaltende Rohphthalsäureanhydrid bei einer Temperatur von 180-280° C mit einem ungesättigten aliphatischen, durch Polymerisation von 1,3-Dienen hergestellten, ggf. isomerisierten Öl vom Molgewicht 800-5000 (bevorzugt 1000-3000) behandelt wird, ggf. in Gegenwart von 1-40 ppm (vorzugsweise 2-20 ppm) Alkaliionen, bezogen auf Rohphthalsäureanhydrid.

## Claim

Process for the removal of naphthoquinone from phthalic anhydride produced by air oxidation of naphthalene or naphthalene-containing mixtures, characterised in that the crude phthalic anhydride containing naphthoquinone is treated at a temperature of 180-280° C with an unsaturated aliphatic oil of molecular weight 800-5,000 (preferably 1,000-3,000), which has been prepared by polymerisation of 1,3-dienes and may have been isomerised, if appropriate in the presence of 1-40 ppm (preferably 2-20 ppm) of alkali metal ions, relative to crude phthalic anhydride.

## Revendication

Procédé pour éliminer la naphtoquinone d'anhydride phthalique fabriqué par oxydation par l'air de naphthaline ou de mélanges contenant de la naphthaline, caractérisé en ce que l'on traite l'anhydride phthalique brut contenant la naphtoquinone, à une température de 180 à 280° C, avec une huile aliphatique, insaturée, dont le poids moléculaire est de 800 à 5000 (de préférence de 1000 à 3000), éventuellement isomérisée, éventuellement en présence de 1 à 40 ppm (ou mieux de 2 à 20 ppm) d'ions alcalins, calculé sur le poids de l'anhydride phthalique brut.